(19) Europäisches Patentamt
European Patent Office
Office européen des brevets

(11) Publication number: **0 097 891**
**B1**

(12) # EUROPEAN PATENT SPECIFICATION

(45) Date of publication of patent specification: **06.11.85**

(51) Int. Cl.$^4$: **C 07 C 45/81**, C 07 C 47/02, C 07 C 29/14

(21) Application number: **83105942.3**

(22) Date of filing: **16.06.83**

(54) **A method for refining crude aldehyde products.**

(30) Priority: **18.06.82 US 389979**

(43) Date of publication of application:
**11.01.84 Bulletin 84/02**

(45) Publication of the grant of the patent:
**06.11.85 Bulletin 85/45**

(84) Designated Contracting States:
**BE DE FR GB IT NL SE**

(56) References cited:
**EP-A-0 008 767**
**DE-B-1 300 541**
**DE-B-2 912 230**
**FR-A-2 456 720**
**US-A-4 247 486**

(73) Proprietor: **UNION CARBIDE CORPORATION**
**Old Ridgebury Road**
**Danbury Connecticut 06817 (US)**

(72) Inventor: **Bunning, Donald Leroy**
**1117 Shamrock Road**
**Charleston, 25314 West Virginia (US)**
Inventor: **Stanton, David Bruce**
**988 Harmony Lane**
**South Charleston West Virginia 25303 (US)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 097 891 B1

**Description**

This invention is directed to a method for refining a crude butyraldehyde product mixture so as to remove organophosphorus contaminants and organic heavies therefrom. The refined aldehyde is then hydrogenated to produce alcohol.

It is well known in the art to hydrogenate aldehydes in the presence of metal catalysts to produce alcohols. The hydrogen adds onto the carbonyl group of the aldehyde to form the alcohol.

It is also well known that the aldehydes which may be hydrogenated may be derived from conventional oxo processes or aldol condensation methods. For instance commonly popular methods for producing such aldehyde starting materials comprise rhodium catalyzed hydroformylation process such as taught for example by U.S. Patents 3,527,809; 4,148,430 and 4,247,486. Such oxo processes involve the hydroformylation of olefins with carbon monoxide and hydrogen in the presence of a rhodium complex catalyst and free organophosphorus ligand to produce the aldehyde. The resultant aldehyde products are mixtures of normal and isomer (branched-chain) aldehydes corresponding to the olefin starting material and result from adding a carbonyl group (—CHO) at one of the carbon atoms of an ethylenic group (e.g. $—CH{=}CH_2$) of the olefin. For instance, the hydroformylation of propylene produces n-butyraldehyde [$CH_3CH_2CH_2CHO$] and isobutyraldehyde [$CH_3CH(CHO)CH_3$]. In general such hydroformylation processes are preferably designed to produce aldehyde products rich in the normal isomer thereof.

As taught in U.S. Patents 4,148,830 and 4,247,486 such continuous hydroformylation processes inherently produce high boiling liquid aldehyde condensation by-products, e.g. trimers and tetramers which may serve as a solvent for the hydroformylation process, as well as other liquid heavies. Thus a small amount of such higher boilers along with some amount of the free organophosphorus ligand is always invariably contained in the crude aldehyde product obtained even after separating the initial aldehyde product from its lights (e.g. carbon monoxide, hydrogen, alkane by-product, etc.) as in the case of a continuous gas recycle hydroformylation process or after separating the initial aldehyde product from its lights and catalyst containing solution as in the case of a continuous liquid recycle hydroformylation process. Indeed even after separating the lower boiling iso-aldehyde from its higher boiling normal aldehyde counterpart in order to provide only a normal aldehyde product feed for producing higher aldehydes [e.g. 2-ethyl-propylacrolein

$$\begin{array}{l}(CH_3CH_2CH_2CH{=}C{-}CHO)\\ \qquad\qquad\qquad\quad |\\ \qquad\qquad\qquad\quad C_2H_5\end{array}$$

from n-butyraldehyde] by conventional aldol condensation such higher aldehyde products may also contain, in addition to the organic heavies of the aldol condensation, the organophosphorus contaminant present in the normal aldehyde product feed.

Accordingly, heretofore it has been the conventional procedure in the art to refine the crude aldehyde product resulting from such conventional continuous rhodium catalyzed hydroformylation processes by removing at least about 99 weight percent, and more preferably at least about 99.9 weight percent, of the organophosphorus contaminant and as much of said higher boiling heavies as practical contained in such crude aldehyde product mixtures by distillation prior to vaporizing and hydrogenating the aldehyde and prior to employing such n-aldehydes as starting materials for such aldol condensation methods, in order to avoid contamination of the finally desired alcoholic product and/or hydrogenation catalyst.

However there are two major penalties associated with commercially refining the crude aldehyde product via distillation. The first is the very high energy cost required to operate such distillation procedures on a commercial level. Secondly a significant amount of aldehyde compound is converted into heavies during such disillation procedures due to the high temperatures necessary to remove said organic heavies and organophosphorus contaminants. Indeed it is estimated that about 1.2 percent by weight of aldehyde compound may be lost by its own *in situ* conversion to heavies that are also substantially removed during such distillation procedures and such is clearly a significant amount in any commercial operation. Moreover, not only is there this loss of aldehyde converted to heavies, there is also an additional direct loss of some aldehyde that is removed from the distillation refining system along with the liquid higher boilers and the organophosphorus contaminants.

European Patent 0008767 is directed to a heterogeneous vapor phase process for the catalytic hydrogenation of aldehydes to alcohols. A benefit of the hydrogenation process of said EPC 0008767 is said to be the low amounts of "heavies" formation caused by the hydrogenation process. Said PCE 0008767 further teaches that the aldehyde feed may comprise the aldehyde(s) product or a portion of the product mixture of, e.g. a conventional oxo product stream.

U.S. Patent 2,549,416 describes a vapor phase process for hydrogenating crude, semi-refined or refined aldehydes by vaporizing same and passing them together with hydrogen over a catalyst comprising reduced copper plus zinc oxide. Said patent also discloses that higher aldehydes may be purified in the vaporizer by separating them from impurities boiling considerably above the boiling point of the aldehyde

and removing such impurities as a liquid from the bottom of the vaporization zone.

It has now been discovered that it is not necessary to separate the organophosphorus contaminant and organic heavies from a crude butyraldehyde product by distillation refining prior to vaporizing the butyraldehyde in the presence of hydrogen and that such drawbacks associated with distillation refining of the crude butyraldehyde product may be overcome or at least greatly minimized by the process of this invention as explained more fully below.

Thus it is an object of this invention to provide a novel method for refining a crude butyraldehyde liquid product mixture, which comprises vaporizing the butyraldehyde of said liquid product mixture and separating a liquid stream of the organophosphorus contaminant and organic heavies therefrom.

More specifically this invention is directed to a method for refining a crude butyraldehyde liquid product mixture consisting essentially of from 95 to 99.5 weight percent butyraldehyde, 5 to 500 parts per million of an organophosphorus contaminant, the remainder consisting essentially of organic heavies, said method comprising separating the organophosphorus contaminant and organic heavies from the butyraldehyde of said liquid product mixture by vaporizing the butyraldehyde of said liquid product mixture while in the presence of a hydrogen containing gas and removing from the vaporizer (1) a vaporized butyraldehyde-hydrogen containing gas stream and (2) a liquid stream of the organophosphorus contaminant and organic heavies.

The crude butyraldehyde liquid product mixture employed herein may be obtained by any conventional metal (preferably rhodium complex) catalyzed hydroformylation process conducted in the presence of free organic phosphorus ligand. Such oxo processes and the conditions thereof are well known in the art as illustrated by the preferred continuous liquid recycle process of U.S. Patent 4,148,830 and the preferred continuous gas recycle process of U.S. Patent 4,247,486. Such hydroformylation processes in general involve producing aldehydes rich in their normal isomers by reacting an olefin with hydrogen and carbon monoxide gas in a liquid reaction medium which contains a soluble rhodium complex catalyst and at least 10 moles of free organophosphorus ligand per mole of catalytically active rhodium and wherein the reaction conditions comprise (1) a temperature of from 50 to 150°C, preferably 90 to 120°C; (2) a total gas pressure of hydrogen, carbon monoxide and olefin of less than 103.4 bar (1500 psia), preferably less than 27.6 bar (400 psia) and more preferably less than 24.1 bar (350 psia); (3) a carbon monoxide partial pressure of less than 6.9 bar (100 psia), preferably 0.07 to 3.45 bar (1 to 50 psia); and (4) a hydrogen partial pressure of less than 27.6 bar (400 psia), preferably 1.38 to 13.8 bar (20 to 200 psia). In general it is preferred that the amount of free organophosphorus ligand is at least 50 moles, and more preferably at least 100 moles per mole of catalytically active rhodium.

Thus as noted herein above the crude butyraldehyde liquid product mixtures consist essentially of butyraldehydes, organic heavies and some of the free organic phosphorus ligand employed in the oxo process and obtained after separating the initial aldehyde product from its lights (e.g. compounds having boiling points below that of the aldehyde product compounds) in the case of a continuous gas recycle hydroformylation process or after separating the initial aldehyde product from its lights and catalyst containing solution as in the case of a continuous liquid recycle hydroformylation process. Of course it is to be understood that while such crude butyraldehyde liquid product mixtures normally contain both normal (straight-chain) aldehyde and iso (branched-chain) aldehyde and are preferably rich in the normal isomer thereof, if desired, the lower boiling iso-aldehyde may be distilled from said crude liquid reaction mixture leaving only or essentially only the normal isomer in said crude liquid mixture. Illustrative aldehydes include isobutyraldehyde and n-butyraldehyde.

Further, it is to be understood that the organophosphorus contaminant contained in the crude butyraldehyde liquid product mixtures employable herein consists essentially of the organophosphorus compound, and/or its corresponding organophosphorus oxide, which corresponds to the free organic phosphorus ligand employed in the oxo processes discussed above from whence the crude butyraldehyde liquid product mixture starting materials of the process of this invention may be derived, as well as any corresponding alkyl substituted phosphorus compounds and/or their corresponding oxides, that might be present as a result of their *in situ* formation or deliberate use during the oxo process. For instance, it is known as seen by U.S. Patent 4,260,828 and 4,297,239 that during the rhodium-triphenylphosphine complex catalyzed hydroformylation of propylene to produce butyraldehyde in the presence of free triphenylphosphine ligand that propyldiphenylphosphine is formed *in situ*. Moreover, it is known that organophosphorus compounds are susceptible to oxidation to their corresponding oxides and that some oxo processes, such as disclosed, e.g. in U.S. Patent 4,221,743, deliberately employ an oxygen containing gas feed to the oxo process system. Thus the organophosphorus oxides that may be present in the organophosphorus contaminant may be as a result of *in situ* or deliberate oxidation during the oxo process or after the formation of the crude butyraldehyde liquid product mixture starting materials of this invention. Accordingly, illustrative organophosphorus compounds may include any organophosphine or organophosphite heretofore advanced for employment in a conventional oxo process. The more preferred organophosphorus compounds will correspond to those free organophosphorus ligands disclosed, e.g. in U.S. Patents

3,527,809; 4,148,830; 4,221,743; 4,247,486; 4,260,828; 4,283,562; 4,297,239 and U.S. Application Serial No. 293,190 filed August 17, 1981. In general triorganophosphines, especially triphenylphosphine, are the most preferred free organophosphorus ligands employed in the production of aldehydes via rhodium catalyzed hydroformylation (oxo) processes.

Likewise the organic heavies contained in the crude butyraldehyde liquid product mixtures employable herein include any organic solvent and organic by-products having boiling points above that of the aldehyde product compounds of the oxo process and/or aldo condensation from whence said product mixtures are derived such as the liquid aldehyde condensation by-products discussed e.g. in U.S.P. 4,148,830 and other common higher boiler by-products.

As noted above the crude butyraldehyde liquid product mixture employable herein may consist essentially of at least 95 to 99.5 weight percent preferably at least 97 to 99 weight percent butyraldehyde; 5 to 500 parts per million, more commonly 10 to 200 parts per million of organophosphorus contaminant based on the total weight of said liquid product mixture; the remainder of said liquid product mixture consisting essentially of organic heavies.

The refining process of this invention comprises separating an organophosphorus contaminant and organic heavies from the butyraldehyde contained in a crude butyraldehyde liquid product mixture by adding said product mixture to a vaporizer and vaporizing the butyraldehyde contained in said mixture while in the presence of a stream of hydrogen containing gas that is also being added to the vaporizer and removing a vaporized butyraldehyde-hydrogen containing gas stream from the vaporizer as well as collecting a liquid stream containing the non-volatilized organophosphorus contaminant and the non-volatilized organic heavies from the vaporizer. In general it is preferred to spray the crude butyraldehyde liquid product mixture down into a vaporization zone while at the same time passing up into said zone a hydrogen containing gas, said gas containing a portion or all of the hydrogen which is to be used in subsequently hydrogenating the aldehyde after it has been vaporized in the presence of said hydrogen containing gas and has been removed from the vaporizer as a vaporized butyraldehyde-hydrogen containing gas stream and passed to a hydrogenator or converter employed for said hydrogenation of the butyraldehyde to its corresponding alcohol(s). Of course it is obvious that the particular design of the vaporization apparatus is not critical and that any conventional vaporization vessel having suitable means for carrying out the process of this invention may be employed herein. Demister pads are helpful for reducing liquid entrainment. The nonvaporized organophosphorus contaminant and nonvaporized organic heavies present in the liquid product mixture may easily be removed as a liquid stream from the bottom of the vaporization zone e.g. at the same time the vaporized butyraldehyde-hydrogen containing gas stream is being removed from the top of the vaporizer or intermittently, if desired.

The vaporization of the butyraldehyde(s) in the refining process of this invention may take place under such conditions as a temperature in the range of from 40 to 150°C, preferably from 60 to 140°C, and at a pressure in the range of from 2.4 to 11.5 bar (i.e. 20 to 150 psig), preferably from 4.5 to 8 bar (i.e. 50 to 100 psig).

In addition the mole ratio of hydrogen containing gas to butyraldehyde(s) in the vaporizer may be at least about 1 mole of hydrogen per mole of butyraldehyde starting material. While the upper limit of the amount of hydrogen employable herein is not critical and is primarily dependent only upon obvious practical processing considerations, when such small amounts of hydrogen are employed, it is of course obvious that make-up hydrogen which by-passes the vaporizer may be added to the vaporized butyraldehyde-hydrogen containing gas after it leaves the vaporizer in order to obtain the most satisfactory hydrogenation results. Thus while as pointed out above the amount of hydrogen in the vaporizer may be a portion or all of the hydrogen to be employed in the subsequent hydrogenation process, it is generally preferred to employ in the process of this invention hydrogen in the range of from 10 to 50 moles per mole of butyraldehyde starting material for such should be suitable in most instances.

Moreover the term "hydrogen containing gas" as used herein obviously includes both essentially pure hydrogen gas as well as gaseous mixtures containing hydrogen, such as mixtures of hydrogen and inert gases such as nitrogen, carbon dioxide and the like. Likewise it is to be understood that the heat required for vaporization of the butyraldehyde(s) may be supplied by any conventional heat exchange means and/or by employing super heated hydrogen containing gas and such a latter method is generally preferred.

Accordingly it is to be understood that the most optimum conditions of temperature, pressure and ratio of hydrogen feed to butyraldehyde starting material in the process of this invention will of course depend on the particular butyraldehyde starting material employed and such optimum conditions may be easily determined by routine experimentation. In general higher vaporizing pressures will require higher temperatures and lower pressures will require lower temperatures.

In general it is preferred to correlate the temperature, pressure and mole ratio of hydrogen to butyraldehyde starting material in the process of this invention so that at least 50 percent by weight, preferably at least 70 percent by weight of the organophosphorus contaminant present in the crude butyraldehyde liquid product mixture to be added to the vaporizer is removed, via the liquid steam of organophosphorus contaminant and organic heavies, from the bottom of the

vaporization zone. Conversely, for any particular combination of such conditions as the particular type of butyraldehyde, vaporizer pressure and mole ratio of hydrogen to butyraldehyde, the preferred temperature for the vaporization will be that temperature at which essentially all of the butyraldehyde is vaporized, but not so low as to have excessive butyraldehyde lost in the liquid tails removed from the vaporizer, nor so high as to vaporize excessive amounts of the organophosphorus contaminant and higher boiling organic heavies of the liquid product mixture starting material. A simple means for determining the preferred temperature for any combination of remaining operating conditions is by analyzing the butyraldehyde concentration in the liquid tails removed from the vaporizer. Preferably the butyraldehyde concentration in said liquid tails should range from 3 to 65 weight percent and more preferably be from 5 to 50 weight percent of the total amount of said liquid tails. Such an butyraldehyde concentration in said liquid tails should correspond in most instances to the preferred process of this invention, i.e. one that has removed at least 50 percent by weight, preferably 70 percent by weight of the organophosphorus contaminant present in the crude butyraldehyde liquid product mixture to be added to the vaporizer via the liquid stream (tails) recovered from the bottom of the vaporization zone. Of course removal of said organophosphorus contaminant in said liquid tails obviously also indicates a substantial removal of the organic heavies present in the liquid product mixture as well.

In accordance with the process of this invention butyraldehyde is preferably vaporized at a temperature ranging from 80 to 95°C and a pressure of about 5.15 bar (60 psig).

The vaporized butyraldehyde-hydrogen containing gas stream removed from the vaporizer of the process of this invention may be hydrogenated to produce alcohol by any conventional hydrogen process. In general it is preferred to hydrogenate said vaporized butyraldehyde-hydrogen containing gas stream by passing it to a converter or hydrogenating vessel and hydrogenating the butyraldehyde by the catalytic hydrogenation process disclosed and described in said above-mentioned European Patent Specification No. 0008767.

Said hydrogenation process comprises contacting the vaporized butyraldehyde-hydrogen containing gas stream produced by the process of this invention with a solid catalyst comprising a reduced mixture of CuO and ZnO. The mole ratio of contained hydrogen gas to butyraldehyde(s) may be generally from 10 to 50 and the hydrogenation process may be conducted at a temperature of between 110 and 180°C preferably between 130 and 170°C and at a pressure of between 2,4 and 11,5 bar (20 and 150 psig), preferably between 4,5 and 8 bar (50 and 100 psig). Moreover, the hydrogenation process is preferably carried out at a space velocity of between 500 and 4000 $hr^{-1}$ (space velocity is total volume of butyraldehyde and hydrogen at standard conditions of temperature and pressure per volume of the catalyst per hour).

The hydrogenation process is most conveniently carried out in a continuous manner, although semi-continuous or batch operations may also be employed. The reaction zone advantageously is an elongated tubular reactor wherein the catalyst is positioned. The vapors are contacted with the catalyst.

The alcohol product from the hydrogenation reaction may be separated from the hydrogen by condensation and the excess hydrogen may be compressed and recycled to the reaction zone. The crude alcohol product may be used in this form or it can be further purified in a conventional manner such as, by fractional distillation. If desired, any unconverted portion of the butyraldehyde or butyraldehyde mixture may be separated from the reaction product and recycled to the reaction zone and preferably, admixed with fresh feed gases prior to entering the reaction zone.

The refining process of this invention is indeed unique in that it provides for not only a very high energy cost savings due to the elimination of such above-described heretofore conventional distillation procedures, but also eliminates or at least greatly minimizes the above-discussed loss in aldehyde due to its *in situ* conversion to heavies that is attendant with such distillation procedures. Moreover, while it might be arguably maintained that one might expect that the organophosphorus contaminant and organic heavies may be separated in such a vaporization procedure, it is equally maintainable that one might also expect some of the organophosphorus contaminant and organic heavies, due to their large amounts in the crude butyraldehyde liquid product mixture to be carried along with the vaporized butyraldehyde-hydrogen containing gas stream removed from the vaporizer and thus adversely affect the performance of the hydrogenation catalyst in terms of the yield of alcohol produced and/or purity of the alcohol product of the subsequent hydrogenation process. However, it has been surprisingly found that the yield and purity of an alcohol product produced by employing a vaporized butyraldehyde-hydrogen containing gas stream as produced by the process of this invention is for all essential purposes substantially the same if not better than that produced from a comparative vaporized butyraldehyde-hydrogen containing gas stream in which the crude butyraldehyde liquid product mixture had undergone a conventional distillation procedure as described herein above to remove the organophosphorus contaminant and organic heavies prior to vaporization of the butyraldehyde. In addition, it is considered that any degradation of the life of the hydrogenation catalyst that might be caused by employing a vaporized butyraldehyde-hydrogen containing gas stream produced by the process of this invention as the

starting material for the hydrogenation process is more than compensated for by the very high energy cost savings and reduction in aldehyde loss caused by conventional distillation refining as discussed above, that is provided by the process of this invention.

The following examples are illustrative of the present invention and are not to be regarded as limitative. It is to be understood that all of the parts, percentages and proportions referred to herein and in the appended claims are by weight unless otherwise indicated.

Examples

Two crude butyraldehyde liquid product mixtures produced within a 5 month interval of each other from the same commercial continuous gas recycle rhodium complex catalyzed hydroformylation process of propylene to produce butyraldehyde rich in its normal isomer, said process being conducted in the presence of excess free triphenylphosphine ligand, each mixture having had its lights removed in the same manner, and which are labeled crude aldehyde liquid product mixtures (A) and (B) for the purpose of these written Examples, were employed as follows.

Crude aldehyde liquid product mixture (A) was subjected to a conventional distillation refining system using two distillation columns in series to remove its organophosphorus contaminant and organic heavies. Table 1 below illustrates the composition of said crude mixture (A) prior to said distillation, as well as the compositions of the refined aldehyde obtained from each distillation column and the total distillation system tails obtained from the second distillation column.

The data in Table 1 shows that about 1.4 weight percent of the aldehyde in the crude aldehyde liquid product mixture (A) feed to the distillation refining system was lost by being converted *in situ* into organic heavies during said distillation procedure. The total system tails stream of the second distillation column corresponds to about 2.5 weight percent of the aldehyde contained in said crude aldehyde liquid product mixture (A) feed. Said total system tails stream of the second distillation column includes the organic heavies made *in situ* during distillation as well as the organic heavies and the organophosphorus contaminant present in said crude aldehyde liquid product mixture (A) feed, and a small amount of butyraldehyde.

Crude aldehyde liquid product mixture (B), which had not been subjected to any distillation refining procedure designed to remove its organic heavies and organophosphorus contaminant, was employed as obtained and fed directly to a vaporizer and the aldehyde contained therein vaporized at about 90°C, under about 5.15 bar (60 psig) in the presence of hydrogen gas (the mole ratio of hydrogen gas to butyraldehyde contained in said crude aldehyde mixture (B) feed being about 23 to 1).

Table 2 below illustrates the composition of said crude aldehyde mixture (B) feed to the vaporizer as well as the composition of the liquid tails stream of organophosphorus contaminant and organic heavies removed from the bottom of the vaporization zone of said vaporizer. The data shows that the total amount of said liquid bottom tails is equivalent to about 1.3 weight percent of the aldehyde in said crude aldehyde liquid product mixture (B) feed. Such indicates that the process of this invention provided a savings of about 1.2 weight percent of the aldehyde contained in said crude aldehyde mixture (B) feed as compared to the conventional distillation refining process carried out on said crude aldehyde mixture (A) above.

Finally the distilled refined aldehyde product mixture obtained from the first distillation column of the distillation procedure outlined above and carried out on crude aldehyde liquid product mixture (A) was vaporized in substantially the same manner and under substantially the same conditions as the vaporization procedure outlined above that was carried out on crude aldehyde liquid product mixture (B).

Both the vaporized aldehyde-hydrogen stream removed from the vaporizer in the case of distilled refined aldehyde product mixture (A) and the vaporized aldehyde-hydrogen stream removed from the vaporizer in the case of crude aldehyde mixture (B) were then hydrogenated in substantially the same manner and under substantially the same conditions according to the invention procedure of said European Patent Specification No. 0008767 ($CuO \cdot ZnO$ catalyst; peak temperature about 170°C; about 5.15 bar (60 psig); space velocity about 956 $hr^{-1}$) to produce butanol. A comparison of both crude butanol products is given in Table 3 below. Said data indicates that the yield and purity of both crude butanol products is substantially the same.

TABLE 1

| Composition | Crude aldehyde liquid product feed (A) | Refined aldehyde from first distillation column | Refined aldehyde from second distillation column | Distillation refining system tails from second distillation column |
|---|---|---|---|---|
| Iso-butyraldehyde (wt. %) | 7.80 | 9.58 | 0.064 | 0.003 |
| N-butyraldehyde (wt. %) | 91.47 | 89.92 | 99.46 | 24.4 |
| Organic heavies (wt. %) | 0.733 | 0.22 | 0.295 | 74.41 |
| Organophosphorus contaminant (ppm) | 388* | — | — | 15 370** |
| Flow rate kg/hr (lbs/hr) | 15 830 (34 867) | 12 982 (28 595) | 2 497 (5 500) | 400 (880) |

* Calculated based on distillation refining system tails from second distillation column.
** Sum of triphenylphosphine, triphenylphosphine oxide and propyldiphenylphosphine detected.

TABLE 2

| Composition | Crude aldehyde liquid product feed (B) to vaporizer | Liquid bottom tails removed from vaporizer |
|---|---|---|
| Iso-butyraldehyde (wt. %) | 10.95 | 1.08 |
| N-butyraldehyde (wt. %) | 87.45 | 13.66 |
| Organic heavies (wt. %) | 1.30 | 81.80 |
| Free organophosphorus contaminant (ppm) | 16.5** | 5 800* |
| Flow rate kg/hr (lbs/hr) | 5 448 (12 000) | 71 (157) |

* Sum of triphenylphosphine and propyldiphenylphosphine detected.
** Reported detection of triphenylphosphine only. Believe total organophosphorus contaminant in feed was about 90 ppm.

TABLE 3

| Composition | Crude butanol produced from distillation refined aldehyde feed (A) to vaporizer | Crude butanol produced from crude aldehyde feed (B) to vaporizer |
|---|---|---|
| Isobutyraldehyde | — | 0.003 |
| N-butyraldehyde | 0.01 | 0.01 |
| Iso-butanol | 10.60 | 9.46 |
| N-butanol | 88.1 | 89.3 |
| Organic heavies | 0.82 | 0.94 |
| Organophosphorus contaminant (ppm) | Nil* | Nil* |

* Flame photometric detector (lower limit of detection estimated to be about 1.7 ppm).

**Claims**

1. A method for refining a crude butyraldehyde liquid product mixture consisting essentially of from 95 to 99.5 weight percent butyraldehyde, 5 to 500 parts per million of an organophosphorus contaminant, the remainder consisting essentially of organic heavies, characterized by separating the organophosphorus contaminant and organic heavies from the butyraldehyde of said liquid product mixture by vaporizing the butyraldehyde of said liquid product mixture while in the presence of a hydrogen containing gas and removing from the vaporizer (1) a vaporized butyraldehyde-hydrogen containing gas stream and (2) a liquid stream of the organophosphorus contaminant and organic heavies; wherein the vaporization is conducted at a temperature in the range of from 40 to 150°C, a pressure in the range of 2.4 to 11.5 bar (20 to 150 psig) and wherein the mole ratio of $H_2$:butyraldehyde is in the range of 1:1 to 50:1.

2. A process as defined in claim 1, wherein the temperature is in the range of 60 to 140°C.

3. A process as defined in claim 1 or 2, wherein the pressure is in the range of 4.5 to 8 bar (50 to 100 psig).

4. A process as defined in any of claims 1 to 3, wherein the organophosphorus contaminant contains triphenylphosphine.

5. A process as defined in any of claims 1 to 4, wherein said vaporized butyraldehyde-hydrogen containing gas stream is passed to a converter and hydrogenated in the presence of a catalyst comprising a mixture of CuO and ZnO at a temperature of between 110 and 180°C, a pressure of between 2.4 and 11.5 bar (20 and 150 psig), and at a space velocity of between 500 and 4000 $hr^{-1}$.

**Patentansprüche**

1. Verfahren zum Raffinieren eines rohen flüssigen Butyraldehyd-Produktgemisches, das im wesentlichen aus 95 bis 99,5 Gewichtsprozent Butyraldehyd, 5 bis 500 ppm einer Organophosphor-Verunreinigung und im übrigen im wesentlichen aus organischen Schwerbestandteilen besteht, dadurch gekennzeichnet, dass man die Organophosphor-Verunreinigung und die organischen Schwerbestandteile von dem Butyraldehyd des flüssigen Produktgemisches trennt, indem man den Butyraldehyd des flüssigen Produktgemisches in Gegenwart eines Wasserstoff enthaltenden Gases verdampft und aus dem Verdampfer (1) einen verdampften Butyraldehyd-Wasserstoff enthaltenden Gasstrom und (2) einen flüssigen Strom der Organophosphor-Verunreinigung und der organischen Schwerbestandteile abzieht; wobei die Verdampfung bei einer Temperatur im Bereich von 40 bis 150°C, einem Druck im Bereich von 2,4 bis 11,5 bar (20 bis 150 psig) durchgeführt wird und wobei das Molverhältnis $H_2$:Butyraldehyd im Bereich von 1:1 bis 50:1 liegt.

2. Verfahren nach Anspruch 1, worin die Temperatur im Bereich von 60 bis 140°C liegt.

3. Verfahren nach Anspruch 1 oder 2, worin der Druck im Bereich von 4,5 bis 8 bar (50 bis 100 psig) liegt.

4. Verfahren nach irgendeinem der Ansprüche 1 bis 3, worin die Organophosphor-Verunreinigung Triphenylphosphin enthält.

5. Verfahren nach irgendeinem der Ansprüche 1 bis 4, worin der verdampfte Butyraldehyd-Wasserstoff enthaltende Gasstrom zu einem Konverter geführt und in Gegenwart eines Katalysators, der eine Mischung aus CuO und ZnO umfasst, bei einer Temperatur zwischen 110 und 180°C, einem Druck zwischen 2,4 und 11,5 bar (20 und 150 psig) und einer Raumgeschwindigkeit zwischen 500 und 4000 $h^{-1}$ hydriert wird.

**Revendications**

1. Procédé pour raffiner un mélange liquide brut de produits contenant essentiellement 95 à 99,5% en poids de butyraldéhyde, 5 à 500 parties par million d'une impureté organophosphorée, le reste consistant essentiellement en produits organiques lourds, caractérisé par la séparation de l'impureté organophosphorée et des composés organiques lourds du butyraldéhyde dudit mélange liquide de produits par vaporisation du butyraldéhyde contenu dans ledit mélange liquide de produits en présence d'un gaz contenant de l'hydrogène et par décharge de l'appareil de vaporisation (1) d'un courant gazeux contenant de l'hydrogène et du butyraldéhyde vaporisé et (2) d'un courant liquide de l'impureté organophosphorée et des composés organiques lourds; la vaporisation étant conduite à une température comprise dans l'intervalle de 40 à 150°C, à une pression manométrique comprise dans l'intervalle de 2,4 à 11,5 bars (20 à 150 $lb/in^2$) et le rapport molaire $H_2$:butyraldéhyde étant compris dans l'intervalle de 1:1 à 50:1.

2. Procédé suivant la revendication 1, dans lequel la température se situe dans l'intervalle de 60 à 140°C.

3. Procédé suivant la revendication 1 ou 2, dans lequel la pression manométrique se situe dans l'intervalle de 4,5 à 8 bars (50 à 100 $lb/in^2$).

4. Procédé suivant les revendications 1 à 3, dans lequel l'impureté organophosphorée contient de la triphénylphosphine.

5. Procédé suivant l'une quelconque des revendications 1 à 4, dans lequel ledit courant gazeux contenant du butyraldéhyde vaporisé et de l'hydrogène est envoyé à un convertisseur et hydrogéné en présence d'un catalyseur comprenant un mélange de CuO et de ZnO, à une température comprise entre 110 et 180°C, à une pression manométrique comprise entre 2,4 et 11,5 bars (20 à 150 $lb/in^2$) et à une vitesse spatiale de 500 à 4000 $h^{-1}$.